(19) Europäisches Patentamt
European Patent Office
Office européen des brevets

(11) Publication number: 0 313 338 B1

# (12) EUROPEAN PATENT SPECIFICATION

(45) Date of publication of patent specification: 16.03.94

(51) Int. Cl.[5]: C07D 213/30, C09K 19/34

(21) Application number: 88309816.2

(22) Date of filing: 19.10.88

## (54) Optically active-2-biphenylpyridines.

(30) Priority: 21.10.87 JP 265934/87

(43) Date of publication of application:
26.04.89 Bulletin 89/17

(45) Publication of the grant of the patent:
16.03.94 Bulletin 94/11

(84) Designated Contracting States:
CH DE FR GB IT LI NL SE

(56) References cited:
EP-A- 0 194 153

PATENT ABSTRACTS OF JAPAN, vol. 13, no. 79 (C-571)[3427], 22nd February 1989, page 107 C 571; & JP-A-63 267 759 (SEIKO EPSON CORP.) 04-11-1988

PATENT ABSTRACTS OF JAPAN, vol. 13, no. 105 (C-575)[3453], 13th March 1989, page 132 C 575; & JP-A-63 280 064 (SEIKO EPSON CORP.) 17-11-1988

(73) Proprietor: Chisso Corporation
6-32, Nakanoshima 3-chome
Kita-ku
Osaka-shi Osaka-fu(JP)

(72) Inventor: Saito, Shinichi
10-1, Otsutomo-cho
Kanazawa-ku
Yokohama-shi Kanagawa-ken(JP)
Inventor: Ohno, Kouji
10-3, Otsutomo-cho
Kanazawa-ku
Yokohama-shi Kanagawa-ken(JP)
Inventor: Inoue, Hiromichi
10-2, Otsutomo-cho
Kanazawa-ku
Yokohama-shi Kanagawa-ken(JP)
Inventor: Miyazawa, Kazutoshi
12-14, Mutsuura 2-chome
Kanazawa-ku
Yokohama-shi Kanagawa-ken(JP)
Inventor: Ushioda, Makoto
12-16, Kannon 1-chome
Kawasaki-ku
Yokohama-shi Kanagawa-ken(JP)

(74) Representative: Lamb, John Baxter et al
MARKS & CLERK
57-60 Lincoln's Inn Fields
London WC2A 3LS (GB)

EP 0 313 338 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a novel liquid crystal compound and a liquid crystal composition containing the same. More particularly it relates to an optically active-2-biphenylylpyridine useful as a component of ferroelectric liquid crystal compositions and a ferroelectric liquid crystal composition containing the same.

At present, TN (Twisted Nematic) display mode has been most broadly employed for liquid crystal display elements. This TN liquid crystal display has a number of advantages such as low driving voltage, small power consumption, etc. However, it is inferior in the aspect of response rate to emissive display elements such as those of cathode tube display, electroluminescence display, plasma display, etc. A novel TN mode display element having increased a twist angle up to 180 - 270° has also been developed, but its response rate is still inferior. As described above, efforts for various improvements have been made, but a TN mode display element having a high response rate has not yet been realized. However, a novel display mode using ferroelectric liquid crystals which has been recently researched extensively has a possibility of notably improving the response rate (Clark et al; Applied Phys. lett., 30, 899 (1980)). This mode is directed to a method utilizing chiral smectic phases such as chiral smectic C phase (hereinafter abbreviated to SC* phase) exhibiting ferroelectric properties. Phases exhibiting ferroelectric properties are not limited only to SC* phase, but it has been known that chiral smectic F, G, H, I phases and the like exhibit ferroelectric properties.

A number of specific features are required for ferroelectric liquid crystal materials used for ferroelectric liquid crystal display elements practically utilized, but at present there is no single compound which satisfies these specific features so that it is necessary to use a ferroelectric liquid crystal composition obtained by mixing some liquid crystal compounds together or mixing non-liquid crystal compound(s) therewith.

Further, ferroelectric liquid crystal compositions are not limited to those consisting only of ferroelectric liquid crystal compounds, but Japanese patent application laid-open No. Sho 61-19518/1986 (Japanese patent application No. Sho 60-36003/1985) proposes that when at least one compound exhibiting ferroelectric liquid crystal phase is mixed with compound(s) or composition(s) exhibiting achiral smectic C, F, G, H, I phase or the like (hereinafter abbreviated to SC phase or the like) as base substance(s), it is possible to obtain a ferroelectric liquid crystal composition as a whole. Further, it has also been reported that when at least one compound which is optically active but exhibits no ferroelectric liquid crystal phase is mixed with compound(s) or composition(s) exhibiting SC phase or the like as main component(s), the whole is made into a ferroelectric liquid crystal composition (Mol. Cryst. Liq. Cryst. 89, 327 (1982)).

In a summary of these matters, it is seen that when at least one compound having an optical activity, irrespective of whether it exhibits ferroelectric liquid crystal phase, is mixed with a liquid crystal substance exhibiting smectic phase, it is possible to compose a ferroelectric liquid crystal composition. However, it is preferred that such an optically active substance, too, exhibit a liquid crystal phase, and even when it exhibits no liquid crystal phase, it is preferred to be a substance having a structure similar to that of liquid crystal compounds, so to speak a quasi liquid crystal substance.

The object of the present invention is to provide an optically active liquid crystal compound useful as a component constituting ferroelectric liquid crystal compositions and a ferroelectric liquid crystal composition containing the same.

The present invention resides in;

an optically active-2-biphenylylpyridine expressed by the formula

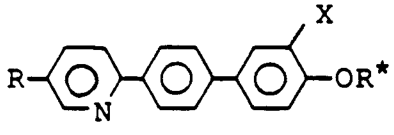

(I)

wherein R represents an alkyl group of 1 to 15 carbon atoms, R* represents an optically active group and X represents hydrogen or fluorine atom, and

a liquid crystal composition containing the same.

Preferred examples of R in the formula (I) are a linear alkyl group of 4 to 12 carbon atoms. Further, preferred examples of R* therein are an optically active alkyl group of 4 to 15 carbon atoms, an optically active halogenated alkyl group of 2 to 13 carbon atoms, an optically active cyanogenated alkyl group of 3 to 14 carbon atoms, an optically active alkoxyalkyl group of 4 to 15 carbon atoms, an optically active halogenated alkoxyalkyl group of 5 to 16 carbon atoms, an optically active alkanoyloxyalkyl group of 5 to 16

carbon atoms, an optically active halogenated alkanoyloxyalkyl group of 5 to 16 carbon atoms, an optically active alkoxyalkanoyloxyalkyl group of 5 to 16 carbon atoms, an optically active alkoxyalkanoyl group of 4 to 15 carbon atoms, an optically active alkanoyl group of 4 to 15 carbon atoms, an optically active halogenated alkanoyl group of 3 to 14 carbon atoms, an optically active alkoxycarbonylalkyl group of 3 to 14 carbon atoms, etc.

Concrete examples of these groups are as follows:

Optically active alkyl group

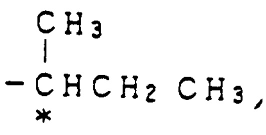 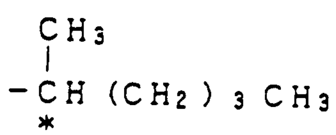 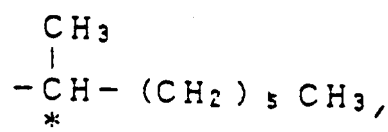

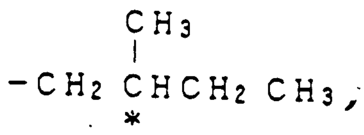 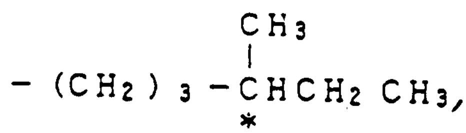 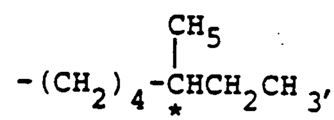

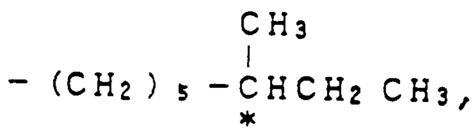 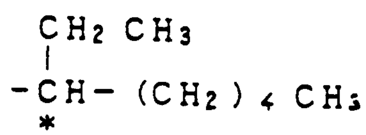 and

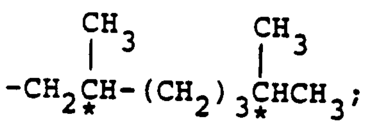

Optically active halogenated alkyl group

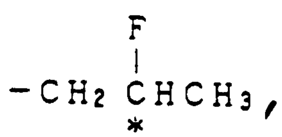 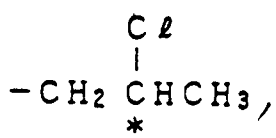 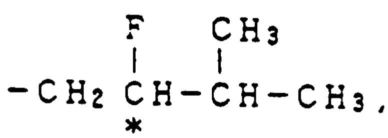

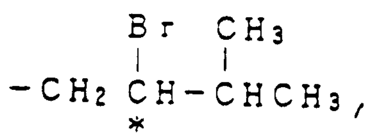 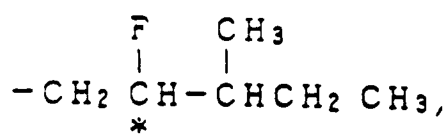 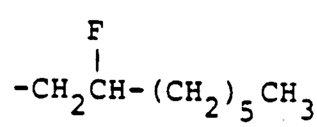

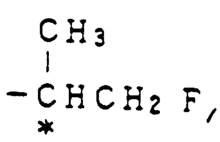 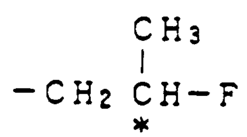 and 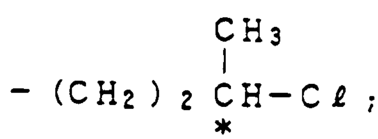

Optically active cyanogenated alkyl group

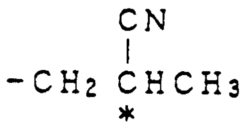
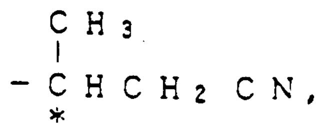
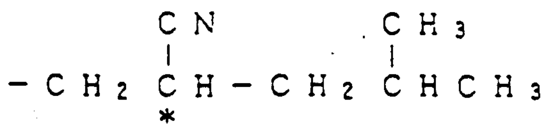

and 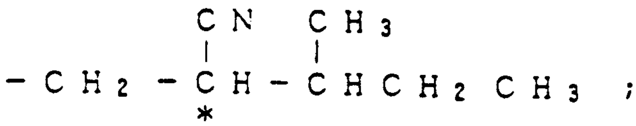

Optically active alkoxyalkyl group

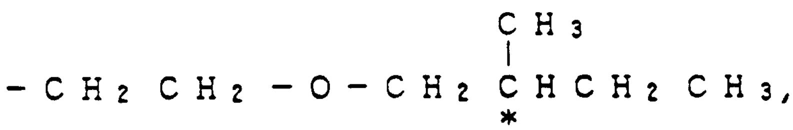

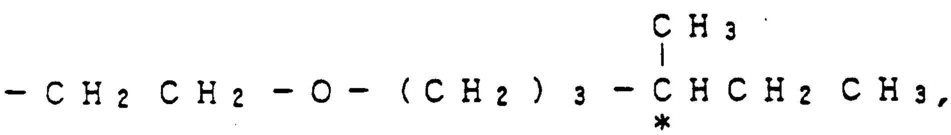

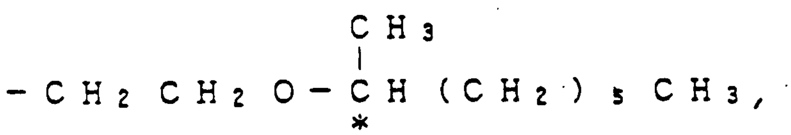

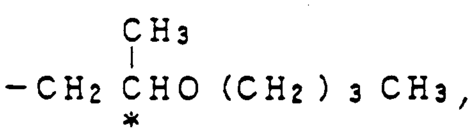
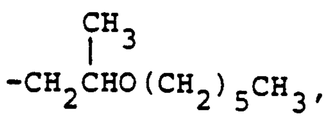

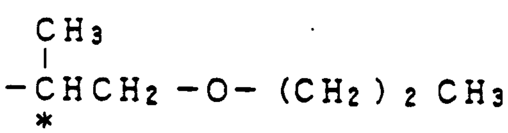 and

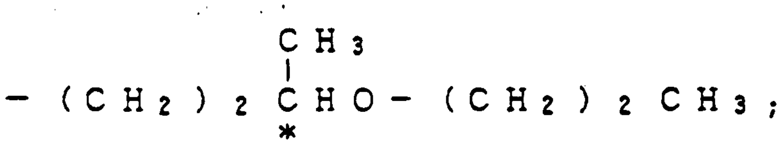

Optically active halogenated alkoxyalkyl group

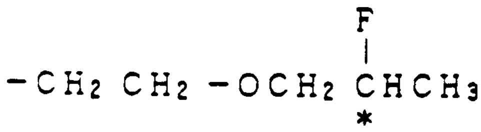 and 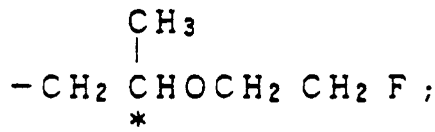

Optically active alkanoyloxyalkyl group

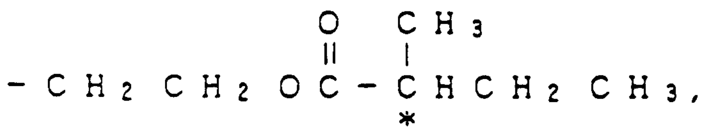

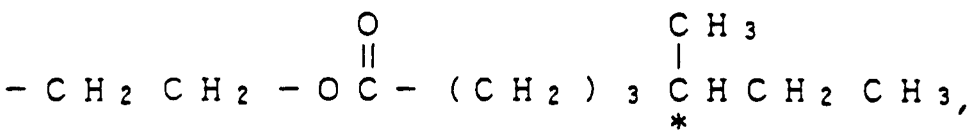

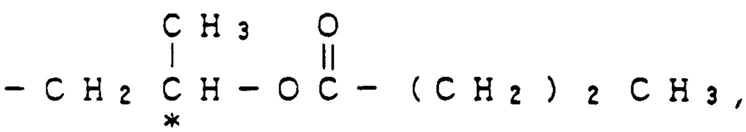

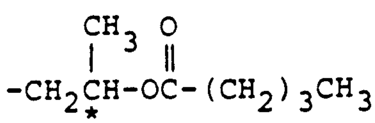 and

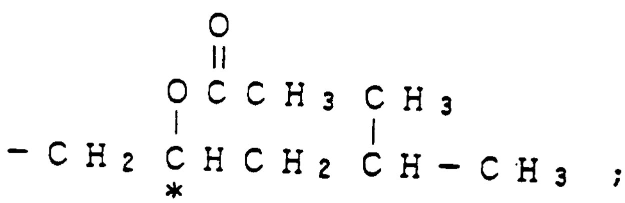

Optically active halogenated alkanoyloxyalkyl group

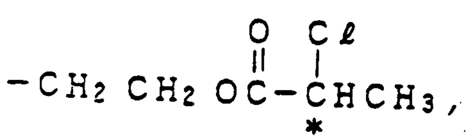 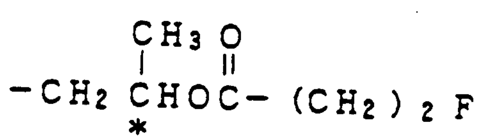

and 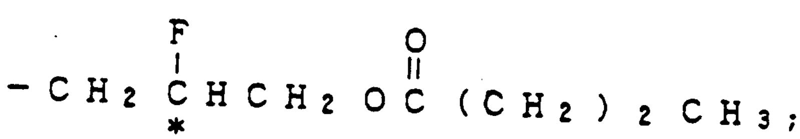

Optically active alkoxyalkanoyloxyalkyl group

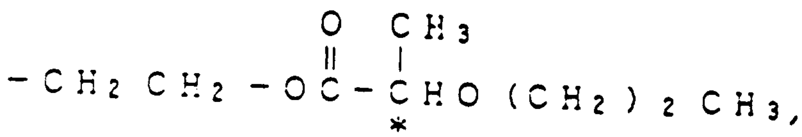

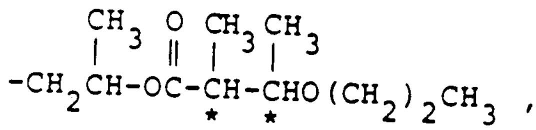

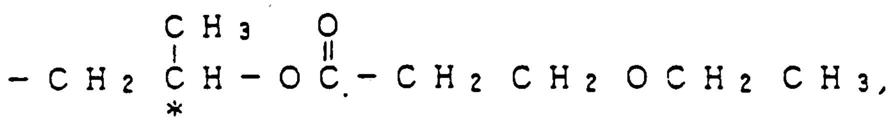

and 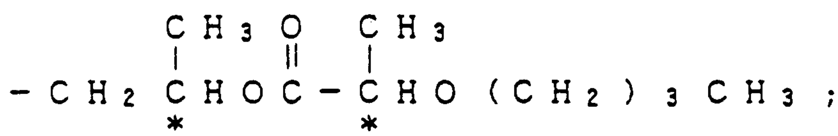

Optically active alkanoyl group

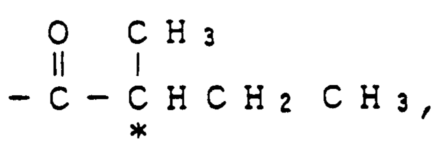

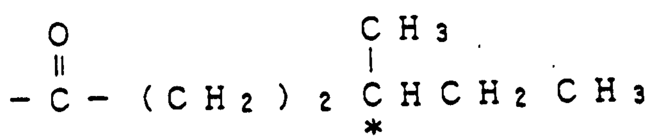

and 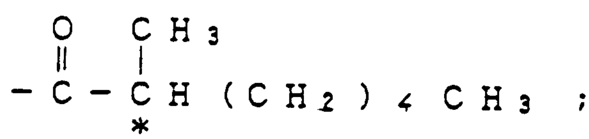

Optically active alkanoyl group

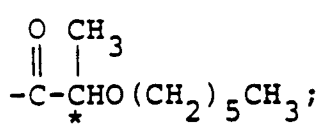

Optically active halogenated alkanoyl group

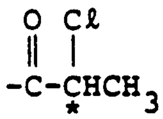 , 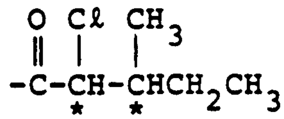 and 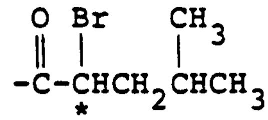 ; and

Optically active alkoxycarbonylalkyl group

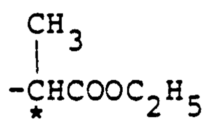 and 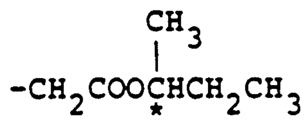.

Representative examples of the compound of the present invention and the phase transition points thereof are show in Table 1. In this Table, the symbol "AC" represents absolute configuration. The symbols C, SA and I are abbreviations of crystalline phase, smectic A phase and Isotropic liquid phase, respectively.

Table 1

| Ex. No. | In formula (I) R | X | R* | AC | Phase transition temperature (°C) C | Sı | SC* | SA | I | Note |
|---|---|---|---|---|---|---|---|---|---|---|
| 1 | -n-$C_4H_9$ | H | 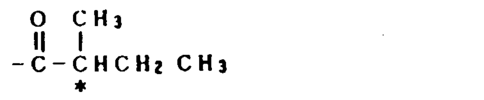 | S | ·103.2 | ·189.4 SG* | —— | —— | · | |
| 2 | -n-$C_4H_9$ | H | 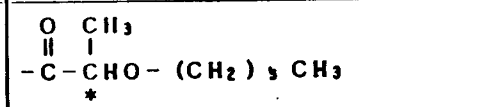 | S | ·152.6 | —— | ·155.7 | ·162.3 | · | Ex. 4 |
| 3 | -n-$C_7H_{15}$ | H | 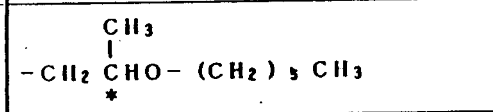 | R | ·52.4 | ·101.0 | ·130.6 | —— | · | |
| 4 | -n-$C_7H_{15}$ | H | 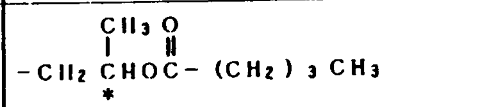 | R | ·77.0 | ·130.8 | ·135.3 | —— | · | |
| 5 | -n-$C_7H_{15}$ | H | 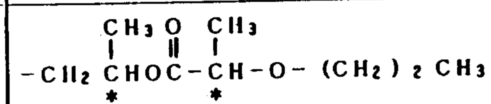 | R,S | ·86.9 | ·100.7 SB | —— | ·114.7 | · | Ex. 1 |
| 6 | -n-$C_8H_{17}$ | H | 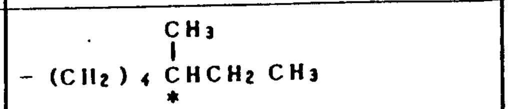 | S | ·unclear | ·168.6 | ·198.2 | —— | · | Ex. 2 |
| 7 | -n-$C_8H_{17}$ | F | 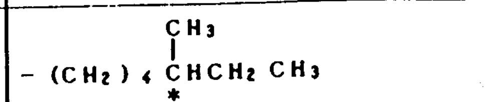 | S | ·75.6 | ·130.6 | ·165.1 | —— | · | Ex. 3 |
| 8 | -n-$C_4H_9$ | H | 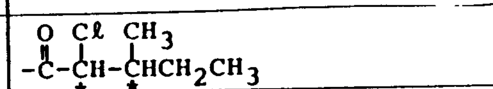 | S,S | ·160.0 | (·157.2) | ·165.1 | ·175.9 | · | |
| 9 | -n-$C_7H_{15}$ | H | 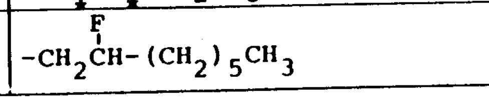 | S | ·122.0 | ·167.0 | ·200.2 | —— | · | |

Many compounds of the present invention exhibit smectic phases, above all tilted smectic phases; hence the compounds are very suitable as a component of ferroelectric liquid crystal compositions. Because ferroelectric properties are exhibited in the respective phases of SC*, SI*, SF*, SG*, SJ*, SH* and SK*, and any of these phases are tilted smectic phases. The properties of the compounds of the present

invention that many compounds exhibit tilted smectic phases are just suitable to ferroelectric liquid crystal display.

Further, the compounds of the present invention include compounds exhibiting a large spontaneous polarization value (hereinafter abbreviated to Ps) which is an important physical property value for ferroelectric liquid crystal materials. Also, the compounds of the present invention include such compounds that although the compounds by themselves exhibit no ferroelectric liquid crystal phase, and no Ps is exhibited , when the compounds are used as a component of ferroelectric liquid crystal compositions, the resulting ferroelectric liquid crystal compositions exhibit a large Ps.

In the case of ferroelectric liquid crystal display, its response rate is proportional to Ps. Large Ps means that a high-speed response is liable to be realized. For example, in the case of the compound of Example 5 of the present application mentioned later, SC* phase exhibiting ferroelectric properties appears at 130.8 °C to 135.3 °C, and the Ps at 132 °C is as very large as 200 nC/cm$^2$. Further, the compound of Example 6 by itself exhibits no ferroelectric liquid crystal phase, but when it is added in 20% to an achiral smectic liquid crystal composition, the resulting liquid crystal composition has a Ps at 25 °C of 15 nC/cm$^2$ (75 nC/cm$^2$ as to the compound, as extrapolated), and a liquid crystal display element having a high-speed response, that is, a response time of 55 $\mu$ sec is obtained.

Further, the compound of the formula (I) of the present invention has an optically active carbon atom; hence when it is added to a nematic liquid crystal, it has a capability of inducing a twist structure. Since a nematic liquid crystal having a twist structure i.e. a chiral nematic liquid crystal does not form the so-called reverse domain of TN mode display elements, the compound of the formula (I) is also usable as an agent for preventing the reverse domain from forming.

Further, when the compound of the formula (I) of the present invention is added to a nematic liquid crystal composition, some of the resulting chiral nematic liquid crystal compositions exhibit a negative temperature-dependency in the chiral pitch length thereof, as shown in Example 8. Most of optically active compounds currently mainly used as an addition to nematic liquid crystal have such a chiral pitch length that the higher the temperature, the larger the length (i.e. a positive temperature-dependency), but it has also been reported that some compounds have a chiral pitch length that the higher the temperature, the less the chiral pitch length (i.e. a negative temperature-dependency), and it has been known that these compounds reduce the temperature-dependency of the threshold voltage as an electrooptical specific feature of TN mode display elements (see The 33rd Applied Physics associated lecture meeting (1986, Spring), lecture preprint 1 p-G-7 (p. 78)) and Japan Display '86, lecture preprint 8.3 (p. 286-289)).

Since the compound of the formula (I) of the present invention has physical properties similar to those of the above-mentioned compounds, it is possible to reduce the temperature-dependency of the threshold voltage of chiral nematic liquid crystal compositions obtained by adding the compound.

Further, apart therefrom, in the case of the so-called super TN mode display having a twist angle increased up to 180° to 270° in the TN mode, the notable temperature-dependency of pitch length remarkably reduces the display quality; thus when a chiral nematic liquid crystal composition obtained by adding the compound of the present invention is used for TN mode display, it is possible to obtain a superior super TN mode display element the display quality of which is not lowered by the temperature change. As described above, the compound of the present invention is also useful as a chiral component compound of chiral nematic liquid crystal compositions.

The compound of the present invention may be prepared through the following route:

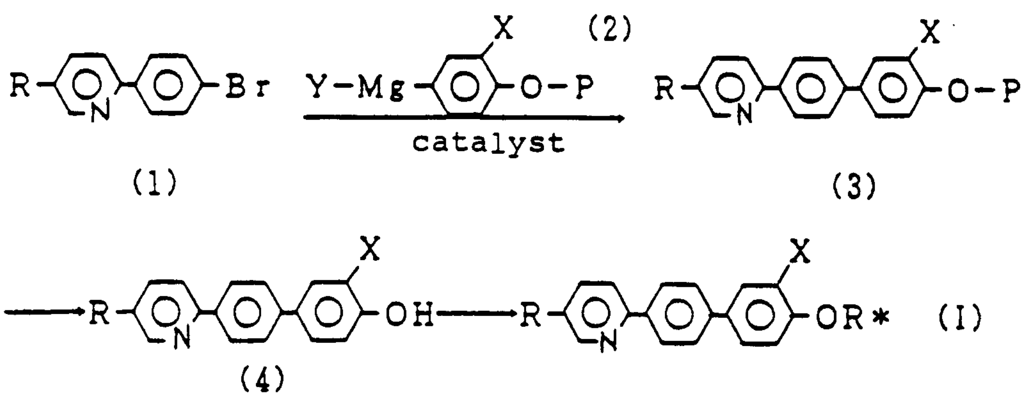


(2)

(1) (3)

(4) (I)

wherein P represents a protecting group, Y represents a halogen atom such as chlorine, bromine, etc. and R, R* and X are as defined above.

Namely, a Grignard reagent (2) is reacted with a 2-(4-bromophenyl)-5-alkylpyridine (1) prepared according to the process disclosed in Japanese patent application laid-open No. Sho 60-163864/1985 in the presence of $NiC\ell_2L_2$ wherein L represents a phosphine ligand, as a catalyst to obtain a compound (3), followed by eliminating the protecting group from the compound in a suitable manner to obtain a phenol (4) and subjecting it to a method according to R* to prepare the compound (I). As the $L_2$ of $NiC\ell_2L_2$, $(PPh_3)_2$, $Ph_2P\text{-}(CH_2)_n\text{-}PPh_2$ (wherein n represents 2 or 3), etc. are particularly preferred.

The compound and composition of the present invention will be described in more detail by way of Examples.

Example 1

Preparation of (2″R,2‴ S)-5-heptyl-2-[4′-{2″-(2‴-propoxypropanoyloxy)propoxy}-4-biphenylyl]pyridine (a compound of the formula (I) wherein R represents $-C_7H_{15}$, R* represents

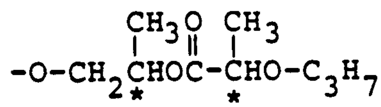

and X represents hydrogen atom) (Sample No. 5)

(First step)

To a solution of 5-heptyl-2-(4′-bromophenyl)pyridine (33.2 g) prepared according to the process described in Japanese patent application laid-open No. Sho 60-163864/1985, dichloro-1,3-bis-(diphenylphosphino)propane nickel (0.5 g) and tetrahydrofuran (hereinafter abbreviated to THF) (100 mℓ) was dropwise added a solution of 4-methoxyphenylmagnesiumbromide (prepared from p-methoxybromobenzene (28.1 g)) in THF (100 mℓ) under cooling, followed by returning the temperature to room temperature and agitating the mixture for 8 hours.

The reaction liquid was then poured in 6N-HCℓ (100 mℓ), followed by adding 2N-NaOH aqueous solution (300 mℓ), carrying out extraction with toluene (300 mℓ), washing the resulting organic layer with water, concentrating it and recrystallizing from ethyl acetate to obtain 5-heptyl-2-(4′-methoxy-4-biphenylyl)-pyridine (17.5 g). This product exhibited liquid crystal phases and the phase transition points were as follows:

$$C \xrightarrow{\text{unclear}} SC \xrightarrow{139°C} N \xrightarrow{212.8°C} I$$

(Second step)

A mixed liquid of 5-heptyl-2-(4′-methoxy-4-biphenylyl)pyridine (17 g) obtained above, hydrobromic acid (48%) (75 mℓ) and acetic acid (170 mℓ) was heated under reflux for 32 hours, followed by allowing the resulting material to cool down to room temperature, adding water (500 mℓ), filtering off the resulting solids, adding them to 2N-NaOH aqueous solution (300 mℓ), dissolving them in the solution on heating, cooling the solution, adding acetic acid, to make pH of the solution 3-4 filtering off the resulting solids, washing them with water and recrystallizing from ethanol to obtain 5-heptyl-2-(4′-hydroxy-4-biphenylyl)pyridine (10 g). M.P.: 183.1 ° C.

(Third step)

THF (50 mℓ) was added to sodium hydride (55% in oil) (0.7 g), followed by adding a solution of 5-heptyl-2-(4′-hydroxy-4-biphenylyl)pyridine (4.0 g) obtained in the second step, in THF (100 mℓ) under

cooling, further adding a dimethylformamide solution (150 mℓ) of (R)-1-(p-toluenesulfonyloxy)-2-(2′-tetrapyranyloxy)propane (5.0 g), heating the mixture at 60° to 80°C for 6 hours, adding toluene (300 mℓ), washing the resulting organic layer with an alkali and then with water, concentrating the layer, purifying the residue according to chromatography using activated alumina (30 g) and toluene as an eluent, again concentrating the elute, adding ethanol (200 mℓ) and conc. hydrochloric acid (5 mℓ), heating the mixture under reflux for 2 hours, distilling off ethanol, adding 2N-NaOH (100 mℓ) to the residue, shaking the mixture, filtering off the resulting solids and recrystallizing from a mixed solution of ethanol (50 mℓ) with ethyl acetate (50 mℓ) to obtain (R)-5-heptyl-2-(4′-2″-hydroxypropoxy-4-biphenylyl)pyridine (2.0 g). This product exhibited liquid crystal properties and its phase transition was as follows:

$$C \xrightarrow{130℃} S_3^* \xrightarrow{173.6℃} S_2^* \xrightarrow{174.3℃} S_1^* \xrightarrow{196.6℃} S_A \xrightarrow{215.8℃} I$$

wherein $S_1$*, $S_2$* and $S_3$* each refer to a ferroelectric liquid crystal phase whose attribution was unknown.

(Fourth step)

(S)-2-propoxypropionic acid (0.6 g) prepared according to the process dislosed in Japanese patent application laid-open No. Sho 63-122650 was added to a solution of (R)-5-heptyl-2-(4′-2″-hydroxypropoxy-4-biphenylyl)pyridine (1.0 g) obtained in the third step, N,N-dicyclohexylcarbodiimide (hereinafter abbreviated to DCC) (1.0 g) and 4-N,N-dimethylaminopyridine (hereinafter abbreviated to DMAP) (0.1 g) in dichloromethane (50 mℓ), followed by agitating the mixture at room temperature for 3 hours, filtering off the resulting solids, washing the filtrate with 2N-NaOH aqueous solution and then with water until the washing water became neutral, concentrating it and recrystallizing the residue from ethanol to obtain the captioned (2″R, 2‴ S)-5-heptyl-2-[4′-{2″-(2″-propoxypropanonyloxy)propoxy}-4-biphenylyl]pyridine (0.6g). This product exhibited liquid crystal phases and the phase transition points were as follows:

$$C \xrightarrow{86.9°C} SB \xrightarrow{100.7°C} SA \xrightarrow{114.7°C} I$$

<u>Example 2</u>

Preparation of (S)-5-octyl-2-{4′-(5″-methylheptyloxy)-4-biphenylyl}pyridine (a compound of the formula (I) wherein R represents $-C_8H_{17}$, R* represents

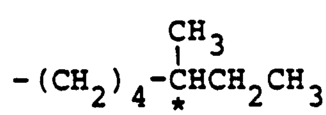

and X represents hydrogen atom) (Sample No. 6)

5-Octyl-2-(4′-hydroxy-4-biphenylyl)pyridine (10 g) prepared in the same manner as in the first and second steps of Example 1 was dissolved in ethanol (100 mℓ), followed by adding KOH (1.8 g), heating the mixture, adding (S)-5-methylheptyl bromide (6.0 g), further refluxing the mixture for 5 hours, concentrating the solution into about 30 mℓ, adding toluene (100 mℓ) to the residue, washing the resulting toluene layer with 2N-NaOH aqueous solution and further with water until the washing water became neutral, concentrating it and recrytallizing from ethanol (150 mℓ) to obtain (S)-5-octyl-2-(4′-(5″-methylheptyloxy)-4-biphenylyl)-pyridine (9.9 g). This product exhibited liquid crystal properties and the phase transition points were as follows:

$$C \overset{\text{unclear}}{\rule{3em}{0.4pt}} S_1 \overset{168.6^\circ C}{\rule{3em}{0.4pt}} SC^* \overset{198.2^\circ C}{\rule{3em}{0.4pt}} I$$

wherein $S_1$ refers to a smectic phase whose attribution was unclear.

Example 3

Preparation of (S)-5-octyl-2-{3′-fluoro-4′-(5″-methylheptyloxy)-4-biphenylyl}pyridine (a compound of the formula (I) wherein R represents $-C_8H_{17}$, R* represents

$$-(CH_2)_4\overset{CH_3}{\underset{*}{C}}HCH_2CH_3$$

and X represents fluorine atom) (Sample No. 7)

Using 5-octyl-2-(3′-fluoro-4′-hydroxy-4-biphenylyl)pyridine in place of 5-octyl-2-(4′-hydroxy-4-biphenylyl)-pyridine used in Example 2 and in the same manner as in Example 2, (S)-5-octyl-2-{3′-fluoro-4′-(5″-methylheptyloxy)-4-biphenylyl}pyridine. This product exhibited liquid crystal phases and the phase transition points were as follows:

$$C \overset{75.6^\circ C}{\rule{3em}{0.4pt}} S_1 \overset{130.6^\circ C}{\rule{3em}{0.4pt}} SC^* \overset{165.1^\circ C}{\rule{3em}{0.4pt}} I$$

Example 4

Preparation of (S)-5-butyl-2-{4′-(2″-hexyloxypropanoyloxy)-4-biphenylyl}pyridine (a compound of the formula (I) wherein R represents $-C_4H_9$, R* represents

$$-\overset{O}{\overset{\|}{C}}-\overset{CH_3}{\underset{*}{C}}HO(CH_2)_5CH_3$$

and X represents hydrogen atom) (Sample No. 2)

(S)-2-hexyloxypropionic acid (8.6 g) was added to a solution of 5-butyl-2-(4′-hydroxy-4-biphenylyl)-pyridine (15 g) prepared in the same manner as in the first and second steps of Example 1, DCC (15 g) and DMAP (2 g) in dichloromethane (100 mℓ), followed by agitating the mixture at room temperature for 3 hours, filtering off the resulting solids, washing the filtrate with 2N-NaOH aqueous solution and then with water, concentrating it and recrystallizing the residue from ethanol (100 mℓ) to obtain (S)-5-butyl-2-{4′-(2″-hexyloxypropanoyloxy)-4-biphenylyl}pyridine (10.1 g). This product exhibited liquid crystal properties and the phase transition points were as follows:

$$C \overset{152.6^\circ C}{\rule{3em}{0.4pt}} SC^* \overset{155.7^\circ C}{\rule{3em}{0.4pt}} SA \overset{162.3^\circ C}{\rule{3em}{0.4pt}} I$$

Example 5

(R)-5-heptyl-2-(4′-2″-pentanoyloxypropoxy-4-biphenylyl)pyridine which is a compound of the present invention (a compound of the formula (I) wherein R represents $-n-C_7H_{15}$, R* represents

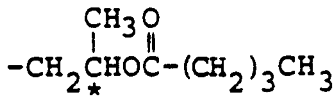

and X represents H) (Sample No. 4 in Table 1) exhibits SC* phase at 130.8° to 135.3°C. This compound had a Ps of 201 (nC/cm$^2$) and a tilt angle of 39.8° at 132.°C. This compound was filled in a cell provided with transparent electrodes each obtained by applying polyvinyl alcohol as an aligning treating agent and rubbing the resulting surface to subject it to a parallel aligning treatment and having a distance between the electrodes of 2 µm, followed by placing the resulting cell between crossed polarizers and impressing a square wave having a wave height of 10 V. As a result, change in the intensity of transmitted light was observed. The response time as measured from the change in the intensity of transmitted light at that time was 20 µ sec at 132°C.

As described above, among the compounds of the present invention, those which singly exhibit ferroelectric liquid crystal phases can compose a liquid crystal display element having a very high response rate within the temperature range.

Example 6

A liquid crystal composition A consisting of

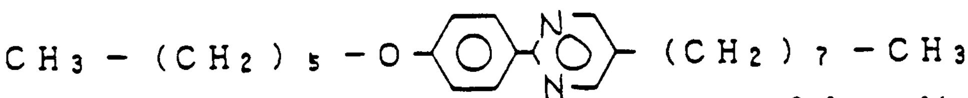

30 wt.%

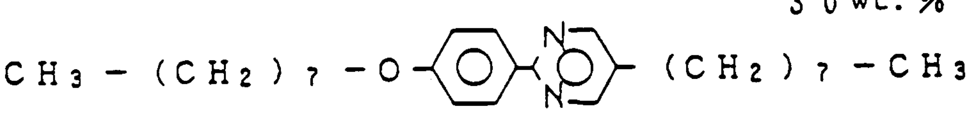

20 wt.%

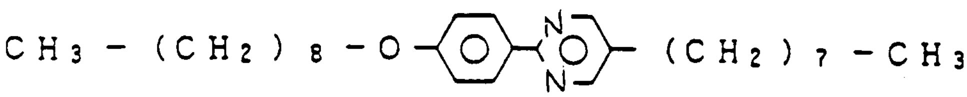

10 wt.%

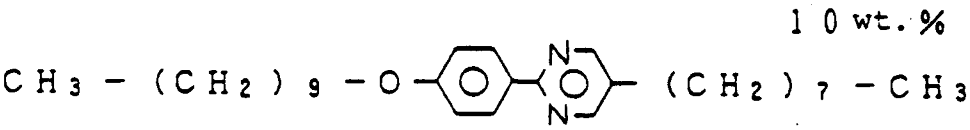

10 wt.%

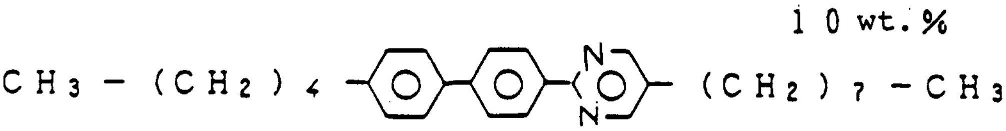

20 wt.% and

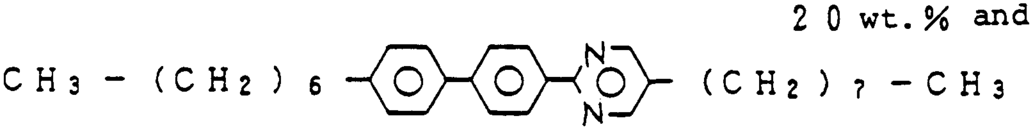

10 wt.%

exhibits a C-SC transition point of 4°C, a SC-SA transition point of 65°C, a SA-Ne transition point of 79°C and a Ne-I transition point of 90°C. Further, since this composition consists of non-optically active compounds, it is not a chiral liquid crystal and hence exhibits no spontaneous polarization.

A mixture a composition B of this composition A (80% by weight) with a compound of Example 1 of the present invention

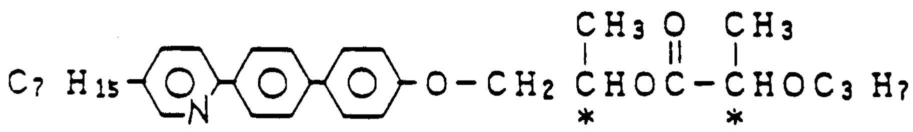 (R,S)

(20% by weight) exhibits a SC*-SA transition point of 53 °C, a SA-Ch transition point of 85.5 °C and a Ch-I transition point of 90.1 °C although its C -SC transition point is unclear. The symbol Ch is an abbreviation of choresteric liquid phase.

The response rate and Ps of this composition B were measured under the same conditions as in Example 5. The results were as follows:

| Temperature | Response time ($\mu$sec) | Ps (nC/cm$^2$) |
|---|---|---|
| 45 | 15 | 10.3 |
| 35 | 30 | 11.7 |
| 25 | 55 | 12.4 |

As described above, when the compound of the present invention was used, it was possible to impart Ps to an achiral smectic liquid crystal composition, and a ferroelectric liquid crystal composition exhibiting a response time of about 50 $\mu$ sec at room temperature was obtained.

Example 7

A nematic liquid crystal composition consisting of

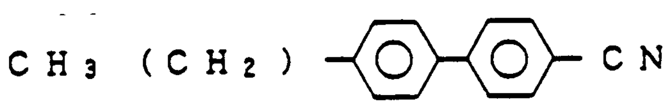 20 wt. %

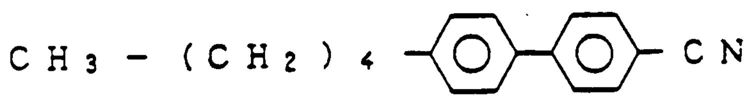 25 wt. %

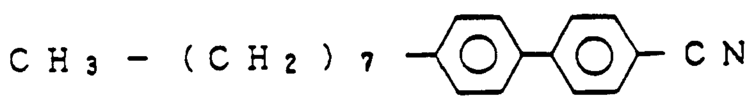 30 wt. % and

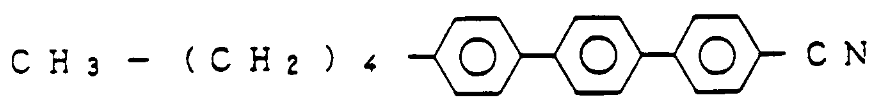 15 wt. %

was filled in a cell provided with transparent electrodes each obtained by applying polyvinyl alcohol as an aligning agent, followed by rubbing the resulting surface to subject it to a parallel aligning treatment and having a distance between the electrodes of 10 $\mu$m to obtain a TN mode display mode and observing it under a polarizing microscope. As a result, a reverse domain was observed to be formed. To this nematic liquid crystal composition was added a compound of the present invention expressed by the formula

(S) 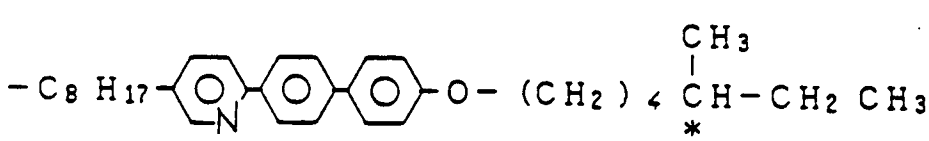

in 1% by weight, followed by preparing a TN mode cell similar to the above from the mixture and observing it. As a result, no reverse domain was formed and a uniform nematic phase was observed.

Example 8

To a commercially available nematic liquid crystal composition (ZLI-1132, a tradename of product made by Merck Company) was added a compound of the present invention expressed by the formula

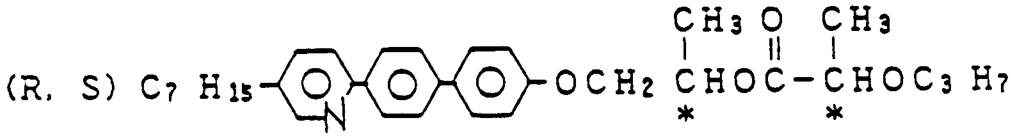

in 1% by weight to prepare a chiral nematic liquid crystal composition. The chiral pitch length of the composition was measured according to Cano-Wedge method. The results were as follows:

| Temp.(°C) | Pitch (μm) |
|---|---|
| 20 | 27.4 |
| 30 | 27.3 |
| 40 | 27.1 |
| 50 | 26.9 |
| 60 | 26.5 |
| 70 | 26.0 |

As seen from the above results, chiral neamtic liquid crystal compositions obtained by adding the compound of the present invention may also include those having a negative temperature-dependency of chiral pitch length.

## Claims

**1.** Optically active-2-biphenylylpyridines of the formula

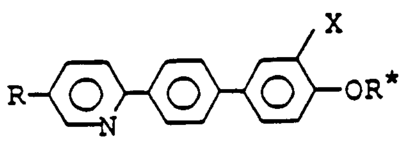

(I)

in which R is a $C_1$-$C_{15}$ alkyl group, R* is an optically active group, and X is a hydrogen or fluorine atom.

**2.** Optically active-2-biphenylylpyridines according to claim 1 in which R is a linear $C_4$-$C_{12}$ alkyl group.

**3.** Optically active-2-biphenylylpyridines according to claim 1 or claim 2 in which R* is an optically active $C_4$-$C_{15}$ alkyl group, an optically active halogenated $C_2$-$C_{13}$ alkyl group, an optically active cyanogenated $C_3$-$C_{14}$ alkyl group, an optically active $C_4$-$C_{15}$ alkoxyalkyl group, an optically active halogenated $C_5$-$C_{16}$ alkanoyloxyalkyl group, an optically active halogenated $C_5$-$C_{16}$ alkanoyloxyalkyl group, an optically active $C_5$-$C_{16}$ alkoxyalkanoyloxyalkyl group, an optically active $C_4$-$C_{15}$ alkanoyl group, an optically active $C_4$-$C_{15}$ aikoxyalkanoyl group, or an optically active $C_4$-$C_{14}$ alkoxycarbonylalkyl group.

**4.** A liquid crystal composition comprising at least two components, at least one of which is an optically active-2-biphenylylpyridine as claimed in any one of the preceding claims.

**5.** A liquid crystal composition according to claim 4, exhibiting a chiral smectic phase.

**6.** A liquid crystal composition according to claim 4, exhibiting a chiral nematic phase.

7. A light switching element comprising a liquid crystal composition as claimed in claim 4.

**Patentansprüche**

1. Optisch aktive 2-Biphenylylpyridine der Formel

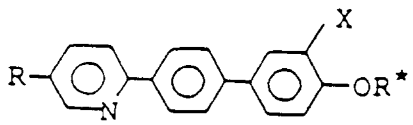

(I)

in welcher R eine $C_1$-$C_{15}$-Alkylgruppe ist, R* eine optisch aktive Gruppe und X ein Wasserstoff oder ein Fluoratom.

2. Optisch aktive 2-Biphenylylpyridine nach Anspruch 1, in welchen R eine lineare $C_4$-$C_{12}$-Alkylgruppe ist.

3. Optisch aktive 2-Biphenylylpyridine nach Anspruch 1 oder 2, in welchen R* eine optisch aktive $C_4$-$C_{15}$-Alkylgruppe, eine optisch aktive halogenierte $C_2$-$C_{13}$-Alkylgruppe, eine optisch aktive mit einem Zyanradikal substituierte $C_3$-$C_{14}$-Alkylgruppe, eine optisch aktive $C_4$-$C_{15}$-Alkoxyalkylgruppe, eine optisch aktive halogenierte $C_5$-$C_{16}$-Alkanoyloxyalkylgruppe, eine optisch aktive halogenierte $C_5$-$C_{16}$-Alkanoyloxyalkylgruppe, eine optisch aktive $C_5$-$C_{16}$-Alkoxyalkanoyloxyalkylgruppe, eine optisch aktive $C_4$-$C_{15}$-Alkanoylgruppe, eine optisch aktive $C_4$-$C_{15}$-Alkoxyalkanoylgruppe oder eine optisch aktive $C_4$-$C_{14}$-Alkoxycarbonylalkylgruppe ist.

4. Flüssigkristallzusammensetzung, welche mindestens zwei Komponenten umfaßt, von denen mindestens eine ein optisch aktives 2-Biphenylylpyridin gemäß einem der voranstehenden Ansprüche ist.

5. Flüssigkristallzusammensetzung nach Anspruch 4, welche eine chirale smektische Phase zeigt.

6. Flüssigkristallzusammensetzung nach Anspruch 4, welche eine chirale nematische Phase zeigt.

7. Lichtschaltendes Element, welches eine Flüssigkristallzusammensetzung gemäß Anspruch 4 umfaßt.

**Revendications**

1. 2-Biphénylylpyridines optiquement actives de la formule:

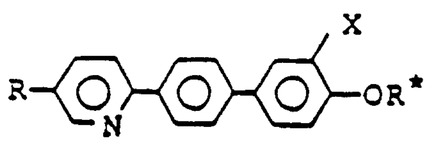

(I)

dans laquelle R est un groupement alkyle en $C_1$-$C_{15}$, R* est un groupement optiquement actif et X est un atome d'hydrogène ou de fluor.

2. 2-Biphénylylpyridines optiquement actives selon la revendication 1, dans laquelle R est un groupement alkyle à chaîne droite en $C_4$-$C_{12}$.

3. 2-Biphénylylpyridines optiquement actives selon la revendication 1 ou la revendication 2, dans laquelle R* est un groupement alkyle en $C_4$-$C_{15}$ optiquement actif, un groupement alkyle halogéné en $C_2$-$C_{13}$ optiquement actif, un groupement cyanoalkyle en $C_3$-$C_{14}$ optiquement actif, un groupement alcoxyalkyle en $C_4$-$C_{15}$ optiquement actif, un groupement alcanoyloxyalkyle halogéné en $C_5$-$C_{16}$ optiquement actif, un groupement alcoxyalcanoyloxyalkyle en $C_5$-$C_{16}$ optiquement actif, un groupement alcanoyle en $C_4$-$C_{15}$ optiquement actif, un groupement alcoxyalcanoyle en $C_4$-$C_{15}$ optiquement actif ou un groupe-

ment alcoxycarbonylalkyle en $C_4$-$C_{14}$ optiquement actif.

4. Composition liquide cristalline comprenant au moins deux composants dont au moins un est une 2-biphénylylpyridine optiquement active telle que revendiquée dans l'une quelconque des revendications précédentes.

5. Composition liquide cristalline selon la revendication 4, présentant une phase smectique chirale.

6. Composition liquide cristalline selon la revendication 4, présentant une phase nématique chirale.

7. Élément de commutation de la lumière comprenant une composition liquide cristalline telle que revendiquée dans la revendication 4.